# EUROPEAN PATENT SPECIFICATION

(11) **EP 4 477 760 B1**
(45) Date of publication and mention of the grant of the patent: **05.11.2025**
(21) Application number: 24173923.4
(22) Date of filing: 02.05.2024
(51) Int. Cl.: C12Q 1/6886

(54) **KIT FOR ASSAYING AFP MRNA FOR LIVER CANCER DIAGNOSIS**
KIT ZUM TESTEN VON AFP-MRNA ZUR LEBERKREBSDIAGNOSE
KIT POUR LE DOSAGE DE L'ARNM DE L'AFP POUR LE DIAGNOSTIC DU CANCER DU FOIE

(30) Priority: 09.06.2023 CN 202310689995
(43) Date of publication of application: 18.12.2024
(73) Proprietor: Sun Yat-Sen University Cancer Center (Affiliated Cancer Hospital of Sun Yat-Sen University, Cancer Research Institute of Sun Yat-Sen University), Guangzhou, 510060 (CN)
(72) Inventor: Yun, Jingping, Guangzhou, 510060 (CN)
(74) Representative: Petraz, Gilberto Luigi

(56) References cited:
- WO-A2-2005/010165
- CN-A- 101 429 550
- CN-A- 109 486 954
- ANONYMOUS: "RNAscopeTM Probe-Hs-AFP", 1 January 2024 (2024-01-01), XP093217297, Retrieved from the Internet <URL:https://acdbio.com/search/site/%2A427411%2A/cms/probes>
- LU SHI-XUN ET AL: "[alpha]-Fetoprotein mRNA in situ hybridisation is a highly specific marker of hepatocellular carcinoma: a multi-centre study", BRITISH JOURNAL OF CANCER, NATURE PUBLISHING GROUP UK, LONDON, vol. 124, no. 12, 6 April 2021 (2021-04-06), pages 1988 - 1996, XP037474216, ISSN: 0007-0920, [retrieved on 20210406], DOI: 10.1038/S41416-021-01363-4
- BREBOROWICZ JAN ET AL: "Detection of Messenger RNAs of a-Fetoprotein and Albumin in a Human Hepatoma Cell Line by in Situ Hybridization", CANCER RESEARCH, vol. 45, no. 4, 1 April 1985 (1985-04-01), pages 1730 - 1736, XP093217287

## Description

### Field

The present disclosure relates to the technical field of molecular biology and, particularly, to a kit for assaying AFP mRNA for liver cancer diagnosis.

### Background

Primary liver cancer is a tumor with high malignancy and poor prognosis. Hepatocellular carcinoma (hereinafter referred as HCC) is the most common histologic type of primary liver cancer, which accounts for 70-90% of primary liver cancers worldwide and is the leading cause of cancer-related deaths worldwide. In China, HCC is the third most common malignant tumor, with a higher incidence in coastal areas than in the interior, and is more common in males. The 5-year survival rate for patients with HCC remains low, which is largely attributed to delayed diagnosis.

Currently, the diagnosis of HCC relies on the patient's clinical manifestations, imaging, serum alpha-fetoprotein assay, and histopathology examination. HCC has a relatively insidious onset and is usually asymptomatic in the early stages, and even remains asymptomatic and atypical in some patients with intermediate to late stages of the disease. Although imaging examinations are localizable, existing studies have found that the presence of large occupancies in the imaging examinations of patients with HCC affects the results, which in turn leads to inaccurate diagnostic results. Histopathological examination is the gold standard for HCC diagnosis. Serum alpha-fetoprotein (AFP) is the main molecular marker for clinical diagnosis and identification of HCC. However, the positive rate of tissue AFP protein assay (immunohistochemical assay) remains low, at only about 30%, and the sensitivity of the assay is relatively low, which limits the application of the assay in the pathological diagnosis of hepatocellular carcinoma. WO 2005/010165 A2 discloses a non-glycosylated human alpha-fetoprotein, methods of production, and uses thereof. CN109486954A discloses a liver cancer diagnosis kit based on AFP mRNA detection.

### Summary

The invention is identified in the appended claims. Provided in the present disclosure is a kit for assaying AFP mRNA for liver cancer diagnosis, the kit including a probe set. The AFP mRNA-specific probes in the probe set are capable of binding specifically to AFP mRNA on tissue slices through complementary pairing in clusters, with high hybridization specificity and short hybridization time, resulting in high positivity rate, high sensitivity and high specificity for liver cancer assay, with accurate results, which is of great significance for the early diagnosis of liver cancer.

In accordance with a first aspect of the present disclosure, provided is a kit for assaying AFP mRNA for liver cancer diagnosis, including a probe set. The probe set includes 20 kinds of the AFP mRNA specific probes, and the nucleotide sequences of the AFP mRNA specific probes are shown in SEQ ID: 1-20, respectively.

In traditional nucleic acid *in situ* hybridization assays and nucleic acid capture assays, a relatively long single-stranded nucleic acid (DNA or RNA) has long been commonly used as a probe, which is hybridized to the nucleic acid to be assayed at a certain temperature and under certain reaction conditions, with the probe usually being modified in a certain manner to facilitate the later assay. However, the preparation and quality control of long-chain probes are costly and unstable, besides, the specificity of long-chain probes is not guaranteed. The non-specific hybridization caused by long-chain probes leads to high background signals, especially when long-chain probes are applied to the assay of RNA in paraffin-embedded tissue slices whose RNA has been significantly degraded, and it is difficult to obtain the signals clearly, which leads to the emergence of false positives, thereby greatly restricting the practical application of these long-chain probes.

The present disclosure is designed for the base sequences between positions 132 and 971 of the AFP mRNA molecule to obtain a number of oligonucleotide probes with lengths ranging from 28 to 44 bp. Due to the short sequence of these oligonucleotide probes, they bind specifically to the base sequences between positions 132 and 971 of AFP mRNA on tissue slices in clusters through complementary pairing, with high hybridization specificity and short hybridization time, which is conducive to the improvement of the sensitivity of the AFP mRNA assay as well as a significant shortening of the assay time. Moreover, these oligonucleotide probes bind to AFP mRNA simultaneously through synergistic action to generate signals, which well avoids excessive background signals or false positives generated by non-specific hybridization of a single probe, thereby enabling more accurate assay of AFP mRNA. In addition, due to the short length of the oligonucleotide probes in the probe set, it allows them to be synthesized and purified by conventional chemical methods with controlled quality and low cost. The probe set obtained from the above design is applied to the kit for assaying AFP mRNA for liver cancer diagnosis, which may be used for the *in situ* assay of AFP mRNA for liver cancer diagnosis, and greatly improves the positivity rate, sensitivity as well as specificity of liver cancer assay, which is of great clinical significance for the early diagnosis of liver cancer.

The probe set in the kit involved in the present disclosure includes 20 oligonucleotide probes (AFP mRNA-specific probes) between 28 and 44 bp in length. These oligonucleotide probes are capable of sequentially binding to the target region in the base region between positions 132 and 691 in the AFP mRNA molecular sequence, with higher hybridization specificity and fewer background signals, with optimal signal-noise ratios of the assay resultant images, which is conducive to the further enhancement of the accuracy of liver cancer assay.

Preferably, AFP mRNA specific probes all include a common primer at a 3' end, and a base sequence of the common primer at the 3' end is shown in SEQ ID: 31.

Preferably, AFP mRNA specific probe is modified by a labeling molecule, the labeling molecule comprising at least one of digoxin, biotin, and horseradish peroxidase.

The 3' end of the AFP mRNA-specific probes in the probe set included in the kit involved in the present disclosure all contain a special binding sequence (TATTATGAGAAAGTTG), to which a specially designed single-stranded DNA (5'→3', CAACTTTCTCATAATA) binds during the signal amplification step. This single-stranded DNA, being modified by the corresponding marker molecules, may be detected in the assay of AFP mRNA for liver cancer diagnosis by the probe set mentioned above by adding to the assay system an antibody/ligand that specifically recognizes these marker molecules or an antibody or ligand labeled with this antibody/ligand by alkaline phosphatase/horseradish peroxidase. Then, a red chromogenic substrate or other substrate for chemical chromogenic reaction is added, and a red/other color insoluble precipitate is formed at the probe binding catalyzed by alkaline phosphatase/horseradish peroxidase, and these granular precipitates may be observed under a microscope, thereby achieving the assay of AFP mRNA for liver cancer diagnosis, with amplified assay signals, which is conducive to the improvement of the sensitivity and accuracy of the assay.

Preferably, the kit further includes an endogenous enzyme inhibitor, the endogenous enzyme inhibitor having a total salt concentration of 0.3 to 0.8 M therein.

The total salt concentration of the endogenous enzyme inhibitor included in the kit involved in the present disclosure is controlled to be between 0.3 and 0.8 M. When the kit is used for the assay of AFP mRNA for liver cancer diagnosis, the background signal may be inhibited in liver samples, which leads to the improvement of the accuracy of the assay.

Preferably, the total salt concentration in the endogenous enzyme inhibitor is 0.6 M.

Preferably, the kit further includes a repair solution, the repair solution including a sodium citrate buffer solution at a concentration of 0.01 M.

Preferably, the kit further includes a digestive solution, the digestive solution including a gastric enzyme at a concentration of 2.5 g/L to 250 g/L.

For most paraffin specimens, employing conventional repair and digestive solutions is only appropriate for samples prepared under fixed standard conditions. However, for liver tissue specimens, especially surgical specimens or punctured tissues that have poor pre-treatment such as mal-fixation, which require better pre-treatment conditions. If these liver tissue specimens are poorly fixed during pre-treatment, the test results may be adversely affected.

The kit involved in the present disclosure optimizes the formulation of the repair solution and digestive solution, which provides a wider sample tolerance than the traditional repair solution and digestive solution, and is suitable for liver samples that do not undergo standard fixed time and fixation method, which also allows the tissue cells to maintain a good cell morphology with no liver samples falling off, so that the tissue cells generate normal positive signals while maintaining their morphology, with a better signal-noise ratio, which leads to a more accurate assay result.

### Brief description of the drawings

Fig. 1 shows a diagram of the staining results of paraffin tissue slices of hepatocellular carcinoma (HCC) and corresponding non-tumorous tissues stained in Example 7 using RNA *in situ* hybridization assay and immunohistochemistry (IHC) staining;
Fig. 2 shows a diagram comparing the diagnostic efficacy of Example 7 for liver cancer using IHC and RNA *in situ* hybridization;
Fig. 3 shows a diagram of the results of RNA *in situ* hybridization assay of AFP mRNA for liver cancer diagnosis in Example 8 using a kit containing endogenous enzyme inhibitors with different total salt concentrations;
Fig. 4 shows a diagram of the results of Example 9 using the kit provided by the present disclosure for assaying AFP mRNA for liver cancer diagnosis on paraffin tissue specimens of different fixation periods.
Fig. 5 shows a diagram of the results of AFP mRNA assay for liver cancer diagnosis in Example 10 using a probe set with different numbers of probes and kits.

### Detailed description of the embodiments

The technical features of the technical solutions in the present disclosure are clearly and completely described below in conjunction with the specific implementations. Obviously, the examples described herein are only some of the examples of the present disclosure but not all of them. Based on the examples in the present disclosure, all other examples obtained by those skilled in the art without creative efforts fall within the scope of protection of the present disclosure.

### Example 1

Provided in the present example is a probe set for assaying AFP mRNA for liver cancer diagnosis, the probe set including 20 probes, the sequences of which are shown in Table 1.

In view of the base sequence between positions 132 and 691 of AFP mRNA (NM_001134.3) for liver cancer diagnosis, the present example is designed to obtain a probe set for assaying AFP mRNA for liver cancer diagnosis. The probe set included 20 AFP mRNA-specific probes, each of which was 44 bp in length, and each of which included a base sequence at the 3' end and a base sequence at the 5' end, with the base sequence at the 3' end used for pairing with the universal nucleic acid and the base sequence at the 5' end used for pairing with the nucleic acid to be assayed, and each of which contained a special binding sequence at the 3' end (5'→3', TATTATGAGAAAGTTG), with the base sequence shown in SEQ ID: 31.

**Table 1 The probe set for assaying AFP mRNA for liver cancer diagnosis provided in Example 1**

| Sequence Number | Base Position | Sequence (5'→3') |
|---|---|---|
| SEQ ID: 1 | 132-159 | tgcagtacattggtaagaatccaatatgTATTATGAGAAAGTTG |
| SEQ ID: 2 | 160-187 | tggtagccaggtcagctaaacttatctcTATTATGAGAAAGTTG |
| SEQ ID: 3 | 188-215 | gcttcttgaacaaactgggcaaaaaataTATTATGAGAAAGTTG |
| SEQ ID: 4 | 216-243 | caccattttgcttacttccttgtaagtgTATTATGAGAAAGTTG |
| SEQ ID: 5 | 244-271 | gtttctcaattgcagtcaatgcatctttTATTATGAGAAAGTTG |
| SEQ ID: 6 | 272-299 | caccctgaagactgttcatctccagtggTATTATGAGAAAGTTG |
| SEQ ID: 7 | 300-327 | cagaaaggcaggtagctggttttctaaaTATTATGAGAAAGTTG |
| SEQ ID: 8 | 328-355 | aaatttctttctcatggcaaagttcttcTATTATGAGAAAGTTG |
| SEQ ID: 9 | 356-383 | cagcagtctgaatgtccgtacttctccaTATTATGAGAAAGTTG |
| SEQ ID: 10 | 384-411 | gttatgtcttccctcttcactttggctgTATTATGAGAAAGTTG |
| SEQ ID: 11 | 412-439 | gagtgggctttttgtgtgcaagaaaacaTATTATGAGAAAGTTG |
| SEQ ID: 12 | 440-467 | ggaacttggaaaagtgggatcgatgctgTATTATGAGAAAGTTG |
| SEQ ID: 13 | 468-495 | atatgcttcacagcttgtgacaggttctTATTATGAGAAAGTTG |
| SEQ ID: 14 | 496-523 | tgttcatgaatgtctccctgtcttcttcTATTATGAGAAAGTTG |
| SEQ ID: 15 | 524-551 | tgccttcttgctatctcataaatgaattTATTATGAGAAAGTTG |
| SEQ ID: 16 | 552-579 | aagaattgtaggtgcatacaggaagggaTATTATGAGAAAGTTG |
| SEQ ID: 17 | 580-607 | ttattttgtcatagcgagcagcccaaagTATTATGAGAAAGTTG |
| SEQ ID: 18 | 608-635 | gcattttcagctttgcagcaagatggaaTATTATGAGAAAGTTG |
| SEQ ID: 19 | 636-663 | tgctgcctttgtttggaagcattcaactTATTATGAGAAAGTTG |
| SEQ ID: 20 | 664-691 | tgctttctcttaattcttttgtaactgtTATTATGAGAAAGTTG |

### Example 2 (comparative example, not covered by the claims)

Provided in the present example is a probe set for assaying AFP mRNA for liver cancer diagnosis, the probe set including 10 probes, the sequences of which are shown in Table 2.

In view of the base sequence between positions 132 and 411 of AFP mRNA (NM_001134.3) for liver cancer diagnosis, the present example is designed to obtain a probe set for assaying AFP mRNA for liver cancer diagnosis. The probe set included 10 AFP mRNA-specific probes, each of which was 44 bp in length, and each of which included a base sequence at the 3' end and a base sequence at the 5' end, with the base sequence at the 3' end used for pairing with the universal nucleic acid and the base sequence at the 5' end used for pairing with the nucleic acid to be assayed, and each of which contained a special binding sequence at the 3' end (5'→3', TATTATGAGAAAGTTG), with the base sequence shown in SEQ ID: 31.

**Table 2 The probe set for assaying AFP mRNA for liver cancer diagnosis provided in Example 2**

| Sequence Number | Base Position | Sequence (5'→3') |
|---|---|---|
| SEQ ID: 1 | 132-159 | tgcagtacattggtaagaatccaatatgTATTATGAGAAAGTTG |
| SEQ ID: 2 | 160-187 | tggtagccaggtcagctaaacttatctcTATTATGAGAAAGTTG |
| SEQ ID: 3 | 188-215 | gcttcttgaacaaactgggcaaaaaataTATTATGAGAAAGTTG |
| SEQ ID: 4 | 216-243 | caccattttgcttacttccttgtaagtgTATTATGAGAAAGTTG |
| SEQ ID: 5 | 244-271 | gtttctcaattgcagtcaatgcatctttTATTATGAGAAAGTTG |
| SEQ ID: 6 | 272-299 | caccctgaagactgttcatctccagtggTATTATGAGAAAGTTG |
| SEQ ID: 7 | 300-327 | cagaaaggcaggtagctggttttctaaaTATTATGAGAAAGTTG |
| SEQ ID: 8 | 328-355 | aaatttctttctcatggcaaagttcttcTATTATGAGAAAGTTG |
| SEQ ID: 9 | 356-383 | cagcagtctgaatgtccgtacttctccaTATTATGAGAAAGTTG |
| SEQ ID: 10 | 384-411 | gttatgtcttccctcttcactttggctgTATTATGAGAAAGTTG |

### Example 3 (comparative example, not covered by the claims)

Provided in the present example is a probe set for assaying AFP mRNA for liver cancer diagnosis, the probe set including 30 probes, the sequences of which are shown in Table 3.

In view of the base sequence between positions 132 and 971 of AFP mRNA (NM_001134.3) for liver cancer diagnosis, the present example is designed to obtain a probe set for assaying AFP mRNA for liver cancer diagnosis. The probe set included 20 AFP mRNA-specific probes, each of which was 44 bp in length, and each of which included a base sequence at the 3' end and a base sequence at the 5' end, with the base sequence at the 3' end used for pairing with the universal nucleic acid and the base sequence at the 5' end used for pairing with the nucleic acid to be assayed, and each of which contained a special binding sequence at the 3' end (5'→3', TATTATGAGAAAGTTG), with the base sequence shown in SEQ ID: 31.

**Table 3 The probe set for assaying AFP mRNA for liver cancer diagnosis provided in Example 3**

| Sequence Number | Base Position | Sequence (5'→3') |
|---|---|---|
| SEQ ID: 1 | 132-159 | tgcagtacattggtaagaatccaatatgTATTATGAGAAAGTTG |
| SEQ ID: 2 | 160-187 | tggtagccaggtcagctaaacttatctcTATTATGAGAAAGTTG |
| SEQ ID: 3 | 188-215 | gcttcttgaacaaactgggcaaaaaataTATTATGAGAAAGTTG |
| SEQ ID: 4 | 216-243 | caccattttgcttacttccttgtaagtgTATTATGAGAAAGTTG |
| SEQ ID: 5 | 244-271 | gtttctcaattgcagtcaatgcatctttTATTATGAGAAAGTTG |
| SEQ ID: 6 | 272-299 | caccctgaagactgttcatctccagtggTATTATGAGAAAGTTG |
| SEQ ID: 7 | 300-327 | cagaaaggcaggtagctggttttctaaaTATTATGAGAAAGTTG |
| SEQ ID: 8 | 328-355 | aaatttctttctcatggcaaagttcttcTATTATGAGAAAGTTG |
| SEQ ID: 9 | 356-383 | cagcagtctgaatgtccgtacttctccaTATTATGAGAAAGTTG |
| SEQ ID: 10 | 384-411 | gttatgtcttccctcttcactttggctgTATTATGAGAAAGTTG |
| SEQ ID: 11 | 412-439 | gagtgggctttttgtgtgcaagaaaacaTATTATGAGAAAGTTG |
| SEQ ID: 12 | 440-467 | ggaacttggaaaagtgggatcgatgctgTATTATGAGAAAGTTG |
| SEQ ID: 13 | 468-495 | atatgcttcacagcttgtgacaggttctTATTATGAGAAAGTTG |
| SEQ ID: 14 | 496-523 | tgttcatgaatgtctccctgtcttcttcTATTATGAGAAAGTTG |
| SEQ ID: 15 | 524-551 | tgccttcttgctatctcataaatgaattTATTATGAGAAAGTTG |
| SEQ ID: 16 | 552-579 | aagaattgtaggtgcatacaggaagggaTATTATGAGAAAGTTG |
| SEQ ID: 17 | 580-607 | ttattttgtcatagcgagcagcccaaagTATTATGAGAAAGTTG |
| SEQ ID: 18 | 608-635 | gcattttcagctttgcagcaagatggaaTATTATGAGAAAGTTG |
| SEQ ID: 19 | 636-663 | tgctgcctttgtttggaagcattcaactTATTATGAGAAAGTTG |
| SEQ ID: 20 | 664-691 | tgctttctcttaattcttttgtaactgtTATTATGAGAAAGTTG |
| SEQ ID: 21 | 692-719 | actgcacatcatgttgatttaacaagcTATTATGAGAAAGTTG |
| SEQ ID: 22 | 720-747 | gaaagttcgggtcccaaaatttttcattTATTATGAGAAAGTTG |
| SEQ ID: 23 | 748-775 | gactcagtttagtaacagttatggcttgTATTATGAGAAAGTTG |
| SEQ ID: 24 | 776-803 | tcagtaaaattaactttggtaaacttctTATTATGAGAAAGTTG |
| SEQ ID: 25 | 804-831 | ggccacatccaggactagtttctggattTATTATGAGAAAGTTG |
| SEQ ID: 26 | 832-859 | ctcctctgcaacagtgctcatgtacatgTATTATGAGAAAGTTG |
| SEQ ID: 27 | 860-887 | tccccatcctgcagacaatccagcacatTATTATGAGAAAGTTG |
| SEQ ID: 28 | 888-915 | ttgagaacatatgtaggacatgatttttTATTATGAGAAAGTTG |
| SEQ ID: 29 | 916-943 | ctattattttgtttgacagagtgtcttgTATTATGAGAAAGTTG |
| SEQ ID: 30 | 944-971 | cgttccagcgtggtcagtttgcagcattTATTATGAGAAAGTTG |

### Example 4

Provided in the present example is a kit for assaying AFP mRNA for liver cancer diagnosis, the kit included:
(1) the probe set provided in Example 1;
(2) an endogenous enzyme inhibitor, including 3% hydrogen peroxide, having a total salt concentration of 0.3 to 0.8 M;
(3) a 10x repair solution, including a sodium citrate buffer solution at a concentration of 0.1 M;
(4) Hybridization Solution 1, Hybridization Solution 2, Hybridization Solution 3, Hybridization Solution 4, and Hybridization Solution 5, in which Hybridization Solution 1 contained 6x sodium citrate buffer (hereinafter referred as SSC), 40 wt% formamide, 50 mg/µL heparin, 1mg/mL tRNA, and 0.2 wt% sodium dodecyl sulfate (hereinafter referred as SDS), in which Hybridization Solution 2 contained 5x SSC, 40 wt% formamide, and 0.2 wt% SDS, in which Hybridization Solution 3 contained 4x SSC, 40 wt% formamide, and 0.3 wt% SDS, in which Hybridization Solution 4 contained 3x SSC, 40 wt% formamide, and 0.2 wt% SDS, in which Hybridization Solution 5 contained 2x SSC, 40 wt% formamide, and 0.3 wt% SDS;
(5) substrate and substrate diluent, the substrate being 3,3-diaminobenzidine (DAB) or 3-Amino-9-ethylcarbazole (AEC), and the substrate diluent including 3% hydrogen peroxide;
(6) 100x digestive solution, which was 250 g/L of gastric enzymes;
(7) 50x wash buffer, which was a TBST buffer, prepared with Tris-HCl, NaCl and Tween-20.

### Example 5 (comparative example, not covered by the claims)

Provided in the present example is a kit for assaying AFP mRNA for liver cancer diagnosis, the kit included:
(1) the probe set provided in Example 2;
(2) an endogenous enzyme inhibitor, including 3% hydrogen peroxide, having a total salt concentration of 0.3 to 0.8 M;
(3) a 10x repair solution, including a sodium citrate buffer solution at a concentration of 0.1 M;
(4) Hybridization Solution 1, Hybridization Solution 2, Hybridization Solution 3, Hybridization Solution 4, and Hybridization Solution 5, in which Hybridization Solution 1 contained 6x sodium citrate buffer (hereinafter referred as SSC), 40 wt% formamide, 50 mg/µL heparin, 1mg/mL tRNA, and 0.2 wt% sodium dodecyl sulfate (hereinafter referred as SDS), in which Hybridization Solution 2 contained 5x SSC, 40 wt% formamide, and 0.2 wt% SDS, in which Hybridization Solution 3 contained 4x SSC, 40 wt% formamide, and 0.3 wt% SDS, in which Hybridization Solution 4 contained 3x SSC, 40 wt% formamide, and 0.2 wt% SDS, in which Hybridization Solution 5 contained 2x SSC, 40 wt% formamide, and 0.3 wt% SDS;
(5) substrate and substrate diluent, the substrate being 3,3-diaminobenzidine (DAB) or 3-Amino-9-ethylcarbazole (AEC), and the substrate diluent including 3% hydrogen peroxide;
(6) 100x digestive solution, which was 250 g/L of gastric enzymes;
(7) 50x wash buffer, which was a TBST buffer, prepared with Tris-HCl, NaCl and Tween-20.

### Example 6 (comparative example, not covered by the claims)

Provided in the present example is a kit for assaying AFP mRNA for liver cancer diagnosis, the kit included:
(1) the probe set provided in Example 3;
(2) an endogenous enzyme inhibitor, including 3% hydrogen peroxide, having a total salt concentration of 0.3 to 0.8 M;
(3) a 10x repair solution, including a sodium citrate buffer solution at a concentration of 0.1 M;
(4) Hybridization Solution 1, Hybridization Solution 2, Hybridization Solution 3, Hybridization Solution 4, and Hybridization Solution 5, in which Hybridization Solution 1 contained 6x sodium citrate buffer (hereinafter referred as SSC), 40 wt% formamide, 50 mg/µL heparin, 1mg/mL tRNA, and 0.2 wt% sodium dodecyl sulfate (hereinafter referred as SDS), in which Hybridization Solution 2 contained 5x SSC, 40 wt% formamide, and 0.2 wt% SDS, in which Hybridization Solution 3 contained 4x SSC, 40 wt% formamide, and 0.3 wt% SDS, in which Hybridization Solution 4 contained 3x SSC, 40 wt% formamide, and 0.2 wt% SDS, in which Hybridization Solution 5 contained 2x SSC, 40 wt% formamide, and 0.3 wt% SDS;
(5) substrate and substrate diluent, the substrate being 3,3-diaminobenzidine (DAB) or 3-Amino-9-ethylcarbazole (AEC), and the substrate diluent including 3% hydrogen peroxide;
(6) 100x digestive solution, which was 250 g/L of gastric enzymes;
(7) 50x wash buffer, which was a TBST buffer, prepared with Tris-HCl, NaCl and Tween-20.

### Example 7

The present example is designed to assay AFP mRNA for liver cancer diagnosis (RNA *in situ* hybridization assay) employing the probe set provided in Example 1 and the kit provided in Example 4, in which a total salt concentration in the endogenous enzyme inhibitors contained in the kit was 0.3 to 0.8 mol/L;
The RNA *in situ* hybridization assay is performed as follows:
(1) Paraffin specimens of fixed-embedded liver tissue were sliced into 4-µm slices, and the resulting slices were baked at 65°C for 1 hour;
(2) Dewaxing: The slices were soaked in fresh xylene for 5 minutes, the xylene-soaked slices were removed and continued to be soaked in fresh xylene for another 5 minutes, then the slices were soaked in fresh anhydrous ethanol for 2 minutes, the ethanol-soaked slices were removed and continued to be soaked in fresh anhydrous ethanol for 2 minutes;
(3) 50x wash buffer was formulated into 1x wash buffer with purified water, 10x repair solution was formulated into 1x repair solution with purified water, and 100x digestive enzyme was formulated into 1x digestive enzyme with PBS buffer;
(4) Three drops of endogenous enzyme inhibitor were added to the slices that had been treated as described above and incubated at room temperature for 30 minutes, then the slices were washed three times with distilled water for 2 minutes each time;
(5) The beaker containing 1x repair solution was heated in advance as a pretreatment solution. After the pretreatment solution was boiled, the slide holder containing the slices was immersed in the 1x repair solution and slowly boiled for 5 minutes, and when the time was up, the slide holder was immediately transferred to a staining vat containing room temperature distilled water and washed 3 times with distilled water for 2 minutes each time, followed by being washed 3 times with fresh anhydrous ethanol for 2 minutes each time, and air-dried;
(6) Water-blocking circles were drawn on the slices with an immunohistochemical pen and washed 3 times with distilled water for 2 minutes each time;
(7) Three drops of 1x digestive solution were added to each specimen in slices and placed in a hybridization oven and incubated at 40°C for 10 minutes;
(8) The probe was preheated at 40°C for 10 minutes, shaken well, and placed at room temperature;
(9) Slices were washed 3 times with distilled water for 2 minutes each time, and each specimen in the slice was added with 3 drops of AFP mRNA probe and incubated in a hybridization oven at 40°C for 2 hours;
(10) The slices were rinsed twice with wash buffer at room temperature for 2 min each time to remove excess liquid, 3 drops of Hybridization Solution 1 were added to each specimen in the section, and the slices were placed in a hybridization oven and incubated at 40°C for 25 min;
(11) The slices were rinsed twice with wash buffer at room temperature for 2 min each time to remove excess liquid, 3 drops of Hybridization Solution 2 were added to each specimen in the section, and the slices were placed in a hybridization oven and incubated at 40°C for 15 min;
(12) The slices were rinsed twice with wash buffer at room temperature for 2 min each time to remove excess liquid, 3 drops of Hybridization Solution 3 were added to each specimen in the section, and the slices were placed in a hybridization oven and incubated at 40°C for 15 min;
(13) The slices were rinsed twice with wash buffer at room temperature for 2 min each time to remove excess liquid, 3 drops of Hybridization Solution 4 were added to each specimen in the section, and the slices were placed at room temperature for 10 min;
(14) The slices were rinsed twice with wash buffer at room temperature for 2 min each time to remove excess liquid, 3 drops of Hybridization Solution 5 were added to each specimen in the section, and the slices were placed at room temperature for 15 min;
(15) The substrate and substrate diluent were prepared in proportion, mixed well and should be used within 10 minutes;
(16) The slices are rinsed twice with wash buffer at room temperature for 2 minutes each time to remove excess liquid, and 3 drops of prepared reaction substrate were added to each specimen in the slice and incubated at room temperature for 5 to 10 minutes;
(17) Hematoxylin staining: The slices were re-stained with hematoxylin for 5-30 seconds, and then the slices were rinsed under running water for 2 minutes;
(18) Bluing: Saturated lithium carbonate was employed to soak the slices for 1 minute, then the slices were rinsed under running water for 10 minutes;
(19) The slices were sealed employing an appropriate amount of sealing adhesive;
(20) Colorful granular precipitates in tissue slices were observed under an ordinary optical microscope, and the positive rate of tissue AFP mRNA was calculated;
(21) The positive rate of AFP mRNA in the enrolled slices of hepatocellular carcinoma, cirrhosis, multiple benign liver tumors, and other malignant tumors was counted, and the positive rate, sensitivity, specificity, and AUC curves of AFP mRNA for the diagnosis of hepatocellular carcinoma were calculated.

Prior to the formal experiment, a pre-experiment was conducted, during which paraffin tissue slices from 10 patients with previously diagnosed liver cancer of various degrees of differentiation were collected and subjected to immunohistochemical staining and RNA *in situ* hybridization assay. The RNA *in situ* hybridization assay refers to assaying AFP mRNA for liver cancer diagnosis using the probe set provided in Example 1 as well as the kit provided in Example 4, and collecting the results of the patient's preoperative serum AFP assay to analyze the correlation of the AFP expression of the three assay techniques. The results showed that the paraffin tissue slices of five patients with positive immunohistochemical staining were all found to be positive by RNA *in situ* hybridization assay, and the paraffin tissue slices in two out of five patients with negative immunohistochemical staining were found to be positive by RNA *in situ* hybridization assay. Paraffin tissue slices from six patients with abnormal serum AFP (>20ng/mL) were all found to be positive by RNA *in situ* hybridization assay, and paraffin tissue slices in one out of four patients with normal serum AFP (≤20ng/mL) were found to be positive by RNA *in situ* hybridization assay.

The cases included in the formal experimental process, the criteria used to include the cases: Patients who had undergone operations at Guangzhou Sun Yat-sen University Cancer Center (SYSUCC) from January 2015 to September 2017 were selected, including 167 cases of hepatocellular carcinoma, 31 cases of cirrhotic nodules, 7 cases of focal nodular hyperplasia, 15 cases of hepatocellular adenomas, 3 cases of heterogeneous hyperplastic nodules, 81 cases of intrahepatic cholangiocarcinomas, and a total of 45 cases of 9 other solid tumors of non-hepatic origin (esophageal cancer, lung cancer, breast cancer, pancreatic cancer, gastric cancer, colorectal cancer, clear cell renal cell carcinoma, and uroepithelial carcinoma), and all patients had not undergone preoperative treatments such as chemotherapy, radiotherapy, and embolization. Paraffin tissue slices of the above cases were collected for conducting formal experiments.

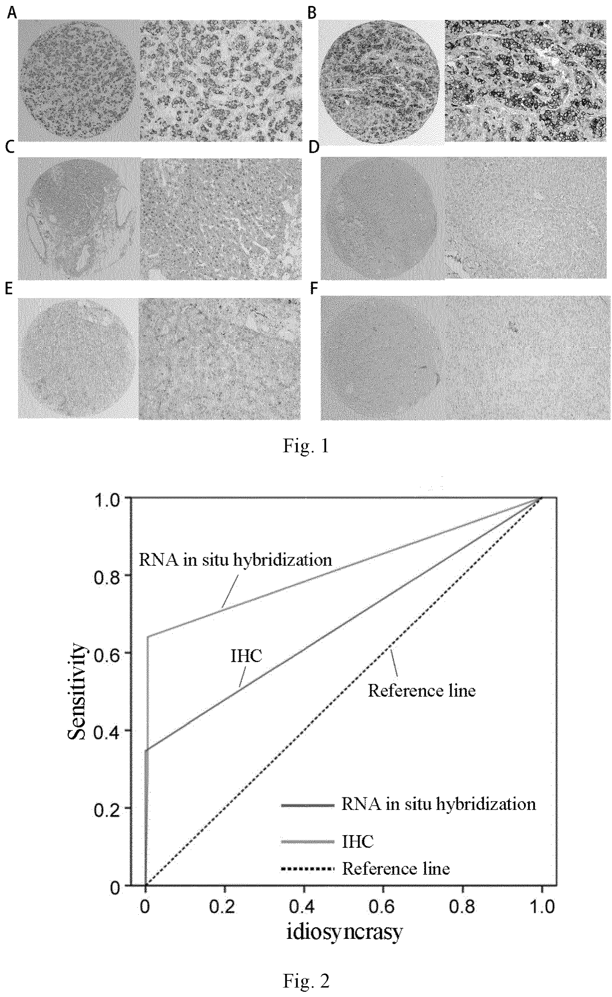

Comparison of the staining of paraffin tissue slices of hepatocellular carcinoma (HCC) and corresponding non-tumorigenic liver tissues was performed by RNA *in situ* hybridization assay of AFP mRNA for liver cancer diagnosis and immunohistochemistry (IHC) staining assay using the probe set provided in Example 1 and the kit provided in Example 4, with the results shown in Fig. 1. The inset A and B of Fig. 1 show positive results by RNA *in situ* hybridization and IHC in the same tumor tissue, respectively, the inset C and D show negative results by RNA *in situ* hybridization and IHC in the corresponding non-tumorous tissues, respectively; and the inset E and F show positive results by RNA *in situ* hybridization and negative results by IHC in the same tumor tissue, respectively.

As shown in Fig. 1, RNA *in situ* hybridization assay of AFP mRNA for liver cancer diagnosis using the probe set provided in Example 1 and the kit provided in Example 4 enables good differentiation of HCC from benign hepatocellular lesions or malignant tumors of non-hepatic origin. Moreover, AFP mRNA was mainly expressed in the cytoplasm of cancer cells and stained with variable intensity, with its scores ranging from 0 to 4 in tumor tissues and negative in non-tumorigenic liver tissues.

Additionally, the expression of AFP in the aforementioned cases was assayed by IHC and RNA *in situ* hybridization. Also, the efficacy of AFP mRNA was compared with that of AFP protein in the diagnosis of HCC, and the results are shown in Table 1 and Fig. 2.

**Table 1 Positive rates of IHC and RNA in situ hybridization in operative samples**

| | Number of incidence N= | Positive Rate | |
|---|---|---|---|
| | | RNA *in situ* hybridization | IHC |
| Hepatocellular carcinoma | 167 | 64.1% | 34.7% |
| Cirrhosis nodule | 31 | 0.0% | 0.0% |
| Focal nodular hyperplasia | 7 | 0.0% | 0.0% |
| Hepatocellular adenoma | 15 | 0.0% | 0.0% |
| Dysplastic nodule | 3 | 0.0% | 0.0% |
| Intrahepatic cholangiocarcinoma | 81 | 0.0% | 0.0% |
| Multiple solid tumors | 45 | 0.0% | 0.0% |

As shown in Table 1 and Fig. 2, according to the results of the ROC curve, the optimal diagnostic threshold for the RNA *in situ* hybridization assay score was 0.5 when the expression of AFP was assayed, and the AFP mRNA expression was up-regulated in HCC, with high specificity, and was not expressed in the rest of the benign liver tumors and other solid tumors. The positive detection rate of AFP protein in liver cancer tissues using IHC was only about 35%, whereas the positive detection rate of liver cancer was up to nearly 65% when the probe set provided in Example 1 and the kit provided in Example 4 were used for assaying AFP mRNA for liver cancer diagnosis by RNA *in situ* hybridization, which is an improvement of about 30% over IHC. This indicates good specificity and confirms that assaying AFP mRNA is a good hepatocellular carcinoma detection method, both as a marker for benign and malignant differentiation to discriminate hepatocellular carcinoma from other hepatocellular origin lesions, and to suggest differentiation of hepatocellular origin. Moreover, RNA *in situ* hybridization assay of AFP mRNA for liver cancer diagnosis using the probe set provided in Example 1 and the kit provided in Example 4 (area under the line AUC=0.817, 95% CI 0.769-0.864, sensitivity=64.1%, specificity=100%) was significantly superior to IHC in the differential diagnosis between hepatocellular carcinoma and other tumors (area under the line AUC=0.674, 95% CI 0.616-0.731, sensitivity=34.7%, specificity=100%), and its diagnostic efficacy was improved by about 20%.

The above results indicate that RNA *in situ* hybridization assay of AFP mRNA for liver cancer diagnosis using the probe set for assaying AFP mRNA for liver cancer diagnosis and the kit containing the same provided by the present disclosure showed excellent sensitivity and specificity, with a positive rate significantly higher than that of immunohistochemical assay of AFP for liver cancer tissues and comparable to that of serum AFP, which provides a good diagnostic efficacy in the assay for liver cancer diagnosis.

### Example 8

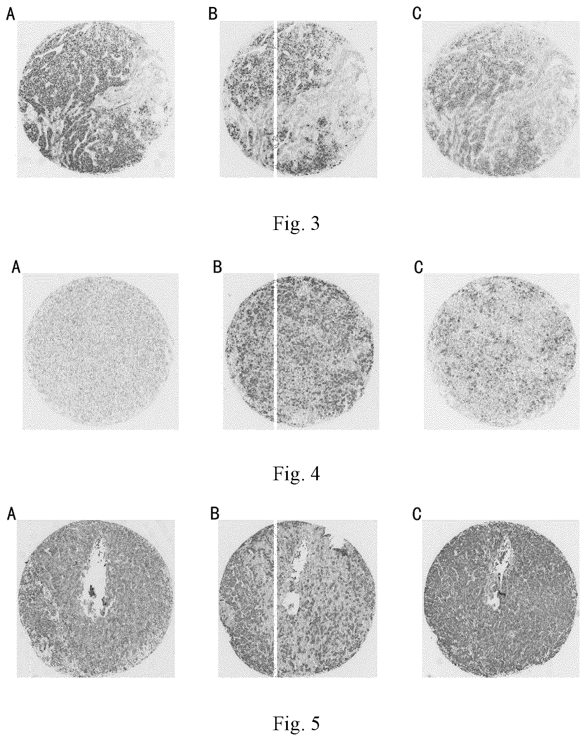

An objective of the present example is to investigate the effect of the total salt concentration in the endogenous enzyme inhibitor in the kit provided by the present disclosure for the assay of AFP mRNA for liver cancer diagnosis on the results of the assay of AFP mRNA for liver cancer diagnosis. RNA *in situ* hybridization assay of AFP mRNA for liver cancer diagnosis was performed using the probe set provided in Example 1 and the kit provided in Example 4, using endogenous enzyme inhibitors with total salt concentrations of 0.1 mol/L, 0.6 mol/L, and 1.2 mol/L, respectively, with reference to the specific operation of the RNA *in situ* hybridization assay in Example 7 for the assay of AFP mRNA for liver cancer diagnosis. The results are shown in Fig. 3. Inset A of Fig. 3 shows the effect of RNA *in situ* hybridization of endogenous enzyme inhibitors with a total salt concentration of 0.1 mol/L, inset B shows the effect of RNA *in situ* hybridization of endogenous enzyme inhibitors with a total salt concentration of 0.6 mol/L, and inset C shows the effect of RNA *in situ* hybridization of endogenous enzyme inhibitors with a total salt concentration of 1.2 mol/L.

As shown in Fig. 3, when the total salt concentration of the endogenous enzyme inhibitor in the kit used for the assay of AFP mRNA for liver cancer diagnosis was 0.1 mol/L, there was a clear background signal in the assay result graph, which affected the accuracy of the assay results (Inset A); when the total salt concentration of the endogenous enzyme inhibitor in the kit used to assay AFP mRNA for liver cancer diagnosis was 1.2 mol/L, the assay signal in the assay result graph was significantly weakened, which leads to inaccurate results and also leads to low positivity rate for liver cancer assay (Inset B); and when the total salt concentration of the endogenous enzyme inhibitor in the kit used to assay AFP mRNA for liver cancer diagnosis was 0.6 mol/L, the assay signal in the assay result graph had the best signal-noise ratio and the most accurate results (Inset C).

The above results demonstrate that when AFP mRNA for liver cancer diagnosis is assayed using the kit for assaying AFP mRNA for liver cancer diagnosis provided by the present disclosure in which the total salt concentration of the endogenous enzyme inhibitor included therein is controlled at a concentration between 0.3 and 0.8 mol/L, the background signal may be inhibited in the liver samples, which leads to the optimal signal-noise ratio of the assayed signals, resulting in a more accurate assay result, thereby increasing the positive rate of liver cancer assay.

### Example 9

An objective of the present example is to investigate the effect on the assay results of the fixation time of the paraffin tissue specimen used in the assay of AFP mRNA for liver cancer diagnosis using the kit for assaying AFP mRNA for liver cancer diagnosis provided by the present disclosure. RNA *in situ* hybridization assay of AFP mRNA for liver cancer diagnosis was performed using the probe set provided in Example 1 and the kit provided in Example 4, and the fixation time of the paraffin tissue specimens used were 6 hours, 18 hours, and 36 hours, respectively, with reference to the specific operation of the RNA *in situ* hybridization assay in Example 7. The results are shown in Fig. 4. Inset A of Fig. 4 shows a diagram of the assay results for a paraffin tissue specimen with a fixation time of 6 hours, inset B shows a diagram of the assay results for a paraffin tissue specimen with a fixation time of 18 hours, and inset C shows a diagram of the assay results for a paraffin tissue specimen with a fixation time of 36 hours.

As shown in Fig. 4, using the probe set provided in Example 1 and the kit provided in Example 4 for RNA *in situ* hybridization assay of AFP mRNA for liver cancer diagnosis, when the fixation time of the paraffin tissue specimen used for the assay is 6 hours, the specimen probe signal is weak; when the fixation time of the paraffin tissue specimen used for the assay is 18 hours, the specimen probe signal is optimal; when the fixation time of the paraffin tissue specimen used for the assay is 36 hours, the specimen probe signal is significantly weaker in comparison with the one of 18 hours.

The above results demonstrate that the kit provided in the present disclosure optimizes the formulation of the repair solution and digestive solution, which provides a wider sample tolerance than the traditional repair solution and digestive solution, and is suitable for liver samples that do not undergo standard fixed time and fixation method, which also allows the tissue cells to maintain a good cell morphology with no liver samples falling off, so that the tissue cells generate normal positive signals while maintaining their morphology, with a better signal-noise ratio in background signal, which leads to a more accurate assay result.

### Example 10

An objective of the present example is to investigate the effect on the assay results of the number of probes in the probe set when the AFP mRNA for liver cancer diagnosis is assayed using the kit for assaying AFP mRNA for liver cancer diagnosis provided by the present disclosure. RNA *in situ* hybridization was performed on AFP mRNA for liver cancer diagnosis using the kits provided in Examples 4 to 6, respectively, with reference to the specific operation in Example 7. The results are shown in Fig. 5. Inset A of Fig. 5 shows a diagram of hybridization results using the kit provided in Example 4 (containing 20 probes) for assaying AFP mRNA for liver cancer diagnosis, inset B shows a diagram of hybridization results using the kit provided in Example 5 (containing 10 probes) for assaying AFP mRNA for liver cancer diagnosis, and inset C shows a diagram of hybridization results using the kit provided in Example 6 (containing 30 probes) for assaying AFP mRNA for liver cancer diagnosis.

As shown in Fig. 5, the kits including 10, 20, and 30 probes were all capable of assaying AFP mRNA for liver cancer diagnosis, all of which achieved hybridization results in line with expectations. However, the signal is relatively weak when 10 probes are employed for assay, and there is obvious background signal when 30 probes are employed for assay, both of which affect the accuracy of the results on assaying AFP mRNA for liver cancer diagnosis to a certain extent, whereas the signal of the probe with 20 probe sequences provides an optimal signal-noise ratio, i.e., the hybridization effect in accordance with the expected results is achieved using the 20 probe sequences, which is the most effective.

The above results demonstrate that the present disclosure is designed for the base sequences between positions 132 and 971 of the AFP mRNA molecule to obtain a number of oligonucleotide probes with lengths ranging from 28 to 44 bp. Due to the short sequence of these oligonucleotide probes, they bind specifically to the base sequences between positions 132 and 971 of AFP mRNA on tissue slices in clusters through complementary pairing, with high hybridization specificity and short hybridization time, which is conducive to the improvement of the sensitivity of the AFP mRNA assay as well as a significant shortening of the assay time. Moreover, these oligonucleotide probes bind to AFP mRNA simultaneously through synergistic action to generate signals, which well avoids background signals or false positives generated by non-specific hybridization of a single probe, thereby enabling more accurate assay of AFP mRNA. When a probe set including 20 oligonucleotide probes of 44 bp length targeting bases between positions 132 and 691 of the AFP mRNA molecule and a kit including them were used for assaying AFP mRNA for liver cancer diagnosis, the oligonucleotide probes continuously bound to the AFP mRNA, leading to a higher hybridization specificity, less background signal, an optimal signal-noise ratio for probe signals, and more accurate assay results.

The above examples are only used to illustrate the technical solution of the present disclosure rather than to limit the scope of the present disclosure Although the present disclosure has been described in detail with reference to the above examples, those skilled in the art should be aware that modifications or equivalent substitutions may be carried out to the technical solution of the present disclosure, but these modifications or substitutions are within the scope of the present disclosure.

## Claims

1. A kit for assaying AFP mRNA for liver cancer diagnosis, comprising a probe set, wherein the probe set comprises 20 kinds of the AFP mRNA specific probes, and the nucleotide sequences of the AFP mRNA specific probes are shown in SEQ ID: 1-20, respectively.

2. The kit for assaying AFP mRNA for liver cancer diagnosis according to claim 1, wherein the AFP mRNA specific probes all comprise a common primer at a 3' end, and a base sequence of the common primer at the 3' end is shown in SEQ ID: 31.

3. The kit for assaying AFP mRNA for liver cancer diagnosis according to claim 1, wherein the AFP mRNA specific probe is modified by a labeling molecule, the labeling molecule comprising at least one of digoxin, biotin, and horseradish peroxidase.

4. The kit for assaying AFP mRNA for liver cancer diagnosis according to any one of claims 1-3, wherein the kit further comprises an endogenous enzyme inhibitor, the endogenous enzyme inhibitor having a total salt concentration of 0.3 to 0.8 M therein.

5. The kit for assaying AFP mRNA for liver cancer diagnosis according to claim 4, wherein the total salt concentration in the endogenous enzyme inhibitor is 0.6 M.

6. The kit for assaying AFP mRNAfor liver cancer diagnosis according to any one of claims 1-3, wherein the kit further comprises a repair solution, the repair solution comprising a sodium citrate buffer solution at a concentration of 0.01 M.

7. The kit for assaying AFP mRNA for liver cancer diagnosis according to any one of claims 1-3, wherein the kit further comprises a digestive solution, the digestive solution comprising a gastric enzyme at a concentration of 2.5 g/L to 250 g/L.

## Patentansprüche

1. Kit zum Testen von AFP-mRNA zur Leberkrebsdiagnose, umfassend einen Sondensatz, worin der Sondensatz 20 Arten der spezifischen AFP-mRNA Sonden umfasst, und die Nukleotidsequenzen der spezifischen AFP-mRNA Sonden jeweils in SEQ ID: 1-20 gezeigt sind.

2. Kit zum Testen von AFP-mRNA zur Leberkrebsdiagnose nach Anspruch 1, worin die spezifischen AFP-mRNA Sonden alle einen gemeinsamen Primer an einem 3'-Ende umfassen, und eine Basensequenz des gemeinsamen Primers an dem 3'-Ende in SEQ ID: 31 gezeigt ist.

3. Kit zum Testen von AFP-mRNA zur Leberkrebsdiagnose nach Anspruch 1, worin die spezifische AFP-mRNA Sonde von einer Markierungsmoleküle geändert ist, wobei die Markierungsmoleküle zumindest eines bzw. eine von Digoxin, Biotin und Meerrettichperoxidase umfasst.

4. Kit zum Testen von AFP-mRNA zur Leberkrebsdiagnose nach einem der Ansprüche 1-3, worin der Kit ferner einen endogenen Enzymhemmer umfasst, wobei der endogene Enzymhemmer eine gesamte Salzkonzentration von 0.3 bis 0.8 M darin aufweist.

5. Kit zum Testen von AFP-mRNA zur Leberkrebsdiagnose nach Anspruch 4, worin die gesamte Salzkonzentration im endogenen Enzymhemmer 0.6 M ist.

6. Kit zum Testen von AFP-mRNA zur Leberkrebsdiagnose nach einem der Ansprüche 1-3, worin der Kit ferner eine Reparaturlösung umfasst, wobei die Reparaturlösung eine Natriumcitrat-Pufferlösung bei einer Konzentration von 0.01 M umfasst.

7. Kit zum Testen von AFP-mRNA zur Leberkrebsdiagnose nach einem der Ansprüche 1-3, worin der Kit ferner eine Verdauungslösung umfasst, wobei die Verdauungslösung ein gastrisches Enzym bei einer Konzentration von 2.5 g/L bis 250 g/L umfasst.

## Revendications

1. Kit pour le dosage de l'ARNm de l'AFP pour le diagnostic du cancer du foie, comprenant un ensemble de sondes,
dans lequel l'ensemble de sondes comprend 20 types de sondes spécifiques de l'ARNm de l'AFP, et les séquences nucléotidiques des sondes spécifiques de l'ARNm de l'AFP sont présentées dans la SEQ ID: 1-20, respectivement.

2. Kit pour le dosage de l'ARNm de l'AFP pour le diagnostic du cancer du foie selon la revendication 1, dans lequel les sondes spécifiques de l'ARNm de l'AFP comprennent toutes une amorce commune à une extrémité 3', et une séquence de base de l'amorce commune à l'extrémité 3' est montrée dans la SEQ ID : 31.

3. Kit pour tester l'ARNm de l'AFP pour le diagnostic du cancer du foie selon la revendication 1, dans lequel la sonde spécifique de l'ARNm de l'AFP est modifiée par une molécule de marquage, la molécule de marquage comprenant au moins l'une de la digoxine, de la biotine et de la peroxydase de raifort.

4. Kit pour le dosage de l'ARNm de l'AFP pour le diagnostic du cancer du foie selon l'une quelconque des revendications 1 à 3, dans lequel le kit comprend en outre un inhibiteur d'enzyme endogène, l'inhibiteur d'enzyme endogène ayant une concentration totale en sel de 0.3 à 0.8 M dans celui-ci.

5. Kit pour le dosage de l'ARNm de l'AFP pour le diagnostic du cancer du foie selon la revendication 4, dans lequel la concentration totale en sel dans l'inhibiteur d'enzyme endogène est de 0.6 M.

6. Kit pour le dosage de l'ARNm de l'AFP pour le diagnostic du cancer du foie selon l'une quelconque des revendications 1 à 3, dans lequel le kit comprend en outre une solution de réparation, la solution de réparation comprenant une solution tampon de citrate de sodium à une concentration de 0.01 M.

7. Kit pour le dosage de l'ARNm de l'AFP pour le diagnostic du cancer du foie selon l'une quelconque des revendications 1 à 3, dans lequel le kit comprend en outre une solution digestive, la solution digestive comprenant une enzyme gastrique à une concentration de 2.5 g/L à 250 g/L.
